# EUROPÄISCHE PATENTSCHRIFT

(11) **EP 0 714 985 B1**
(45) Veröffentlichungstag und Bekanntmachung des Hinweises auf die Patenterteilung: **20.12.2000**
(21) Anmeldenummer: 95117976.1
(22) Anmeldetag: 15.11.1995
(51) Int. Cl.: C12Q 1/00, G01N 27/327

(54) **Elektrochemischer Enzymbiosensor**
Electrochemical enzymatic biosensor
Biosenseur électrochimique enzymatique

(30) Priorität: 28.11.1994 DE 4442253
(43) Veröffentlichungstag der Anmeldung: 05.06.1996
(73) Patentinhaber: Bayer Corporation, Pittsburgh, PA 15205-9741 (US)
(72) Erfinder: Hampp, Norbert, Prof. Dr., D-35287 Amöneburg-Rossdorf (DE); Braeuchle, Christoph, Prof. Dr., D-81739 München (DE); Silber, Anton, D-86415 Mering (DE)
(74) Vertreter: Kirchner, Dietrich, Dr.

(56) Entgegenhaltungen:
- EP-A- 0 289 344
- ANALYTICAL CHEMISTRY, Bd. 47, Nr. 8, Juli 1975 COLUMBUS US, Seiten 1337-1343, XP 000563210 W. J. BLAEDEL & R. A. JENKINS 'Study of the electrochemical oxidation of reduced nicotinamide adenine dinucleotide.'
- ELECTROANALYSIS, Bd. 4, Nr. 1, Januar 1992 Seiten 107-110, XP 000564043 C. HUA ET AL 'Voltammetric behavior of dihydronicotinamide adenine dinucleotide phosphate at enzyme-modified electrodes.'
- JOURNAL OF BIOTECHNOLOGY, Bd. 27, 1993 AMSTERDAM NL, Seiten 129-142, XP 000563736 W. SCHUHMANN ET AL 'Electrocatalytic oxidation of NADH at mediator-modified electrode surfaces.'

## Beschreibung

Die Erfindung geht aus von einem elektrochemischen Enzymbiosensor mit Edelmetallelektroden, bei dem eine elektrochemische Oxidation von Pyridinnukleotiden, insbesondere von NADH erfolgt.

Eine Vielzahl von enzymanalytischen Verfahren der klinischen Diagnostik sowie der Umwelt-, Prozeß- und Lebensmittelanalytik basieren auf Reaktionen, die von Enzymen der Klasse der Dehydrogenasen katalysiert werden. Dabei nutzt man die direkte Proportionalität zwischen der Konzentration des zu analysierenden Substrates einerseits und der Konzentration des gebildeten bzw. verbrauchten Co-Enzyms NADH andererseits. In der Mehrzahl machen sich diese Verfahren optische Eigenschaften des Moleküls NADH zunutze, das sich z.B. durch eine charakteristische Absorption von Licht der Wellenlänge 360 nm von seiner korrespondierenden oxidierten Form NAD⁺ unterscheidet. Im Falle gefärbter oder trüber Analyten versagen diese spektrophotometrischen Methoden weitgehend oder machen zumindest eine arbeits- und zeitaufwendige Probenvorbehandlung notwendig. Diese kann durch elektrochemische Detektion von NADH umgangen werden. Außerdem ist eine direkte, stabile Kopplung von Elektrode und Enzym zur Bildung von elektrochemischen Biosensoren möglich. Der Nachteil der elektrochemischen Methode liegt bisher in der hohen benötigten Überspannung zur Oxidation von NADH. Durch sie werden eine Reihe weiterer Substanzen, die ebenfalls im Analyten vorhanden sind, miterfaßt, so daß normalerweise zu hohe Analysenwerte erhalten werden, die nachträglich korrigiert werden müssen. Außerdem führt die elektrochemische Oxidation von NADH bei hohen Überspannungen über radikalische Zwischenstufen zu Di- und Oligomeren von NADH, die die Elektrodenoberfläche stabil belegen und nachhaltig schädigen (sog. Elektrodenfouling, siehe dazu W.J. Blaedel & R.A. Jenkins, Study of the electrochemical oxidation of reduced nicotinamide adenine dinucleotide, Anal. Chem. 47 (1975) 1337-1343). Um dieses Phänomen zu umgehen, setzt man dem Analyten oftmals katalytische Mengen redoxfähiger Moleküle kleiner Molmasse zu, die den Transport der Elektronen von NADH zur Elektrode bewerkstelligen (siehe dazu u.v.a. L. Gorton, Chemically modified electrodes for the electrocatalytic oxidation of nicotinamide coenzymes, J. Chem. Soc. Faraday Trans. 82 (1986) 1245-1258). Diese sogenannten Mediatoren werden z.T. an die Elektrode immobilisiert, wobei wegen der Einschränkung, daß der Mediator beweglich bleiben muß, um wirksam zu sein, oftmals eine stabile Anbindung des Mediators an die Elektrode nicht möglich ist. Dieser diffundiert in den Analyten, worunter die Stabilität der Sensoren erheblich leidet.

Eine in US-PS 5 240 571 beschriebene Methode verwendet ein redoxfähiges Kopplungsreagenz, das dem Analyten zugesetzt wird und mit NADH eine elektroaktive Verbindung bildet, die bei niedrigen Überspannungen oxidiert wird. Aus US-PS 5 122 456 ist bekannt, daß NADH quantitativ an mit Platin oder Palladium belegten, porösen Kohlenstoff- und Graphitelektroden oxidiert werden kann, wobei das verwendete Potential nicht angegeben wird.

Der Erfindung liegt die Aufgabe zugrunde, einen elektrochemischen Enzymbiosensor mit Edelmetallelektroden, ohne Zusatz eines Mediators zum Analyten und ohne chemische Modifikation der Elektrode derart zu verbessern, daß etwaige im Analyten enthaltene Interferenzsubstanzen, die ebenfalls elektrochemisch oxidierbar sind, weitgehend keine Elektrodenreaktion eingehen können. Neben der dadurch gewonnenen höheren Selektivität soll die Stabilität der Transduktorelektroden und damit die Betriebsstabilität der daraus hergestellten Sensoren erhöht werden.

Diese Aufgabe wird, ausgehend von einem elektrochemischen Enzymbiosensor mit Edelmetallelektroden, bei dem eine elektrochemische Oxidation von Pyridinnucleotiden erfolgt, erfindungsgemäß dadurch gelöst, daß die Edelmetallelektroden eine oberflächenporenreiche Mikrostruktur mit katalytischen Eigenschaften aufweisen, wodurch die für die elektrochemische Oxidation der Pyridinnucleotide notwendige Überspannung herabgesetzt wird. Die oberflächenporenreiche Mikrostruktur ist durch zahlreiche mikroskopisch kleine Spitzen und Krater bedingt, so daß die Oberfläche der Elektroden erheblich vergrößert wird. Die Spitzen bestehen aus Edelmetallpartikel oder aus Partikelclustern, die aus der Elektrodenoberfläche herausragen und einen mittleren Krümmungsradius von 0,05 µm bis 4 µm, vorzugsweise von 0,5 µm bis 2 µm aufweisen. Durch diese Mikrorauhigkeit wird die Oberfläche einer Elektrode etwa um den Faktor 3 bis 10, vorzugsweise 4 bis 7 vergrößert (verglichen mit der geometrischen Oberfläche), was durch elektronenmikroskopische und impedanzspektroskopische Messungen nachgewiesen werden kann

Diese besondere oberflächenreiche Mikrostruktur der Edelmetallelektroden führt dazu, daß die Elektroden katalytische Eigenschaften aufweisen, die sich in einer drastischen Reduktion der Überspannung für die Oxidation von Pyridinnukleotiden insbesondere von NADH, bemerkbar machen. Es wurde gefunden, daß die zur anodischen Oxidation von NADH zu NAD⁺ erforderliche Überspannung auf 0 bis 200 mV, vorzugsweise auf 100 bis 150 mV gegen SCE herabgesetzt wird und damit um mindestens 50 % unter den sonst üblichen Werten liegt.

Besonders gute Ergebnisse werden erzielt, wenn die mikroporösen Edelmetallelektroden aus Gold bestehen.

Die Immobilisierung der selektivitätsbestimmenden Enzyme kann bevorzugt dadurch erfolgen, daß eine das Enzym enthaltende Membran auf die Arbeitselektrode aufgebracht wird. Die Haftung der Membran auf der planaren Elektrode wird durch die oben beschriebene Mikrorauhigkeit der Elektrode begünstigt. Insbesondere mit Membranen auf wäßriger Polyvinylacetatdispersion, wie sie z.B. in DE 40 27 728 beschrieben werden, können stabile, selektive hochempfindliche Biosensoren realisiert werden.

Gemäß einer Weiterentwicklung werden die mikroporösen Edelmetallelektroden durch eine Beschichtung mit elektropolymerisierbaren leitfähigen Polymeren, insbesondere mit Polypyrrol und Polymethylenblau modifiziert. Diese Modifizierung kann zur Immobilisierung von Enzymen u.a. selektivitätsgebenden Biokomponenten herangezogen werden. Die disperse Oberfläche der Edelmetallelektroden ermöglicht dabei ein homogenes Wachstum des Polymers während der Elektropolymerisation, da viele reaktive, gleichmäßig über die gesamte makroskopische Elektrodenoberfläche verteilte Zentren erzeugt werden.

Wird die elektrochemische Polymerisation in Gegenwart eines Enzyms durchgeführt, so wird dieses durch physikalischen Einschluß in das Polymer während dessen Wachstum vor der Elektrode immobilisiert. Das Polymergerüst dient einerseits der Immobilisierung des Enzyms, hat aber auch die Funktion eines Siebes, welches verhindert, daß große Moleküle, z.B. Proteine, an die Elektrodenoberfläche gelangen, diese belegen und damit zumindest reversibel schädigen. Desweiteren kann das Polymer, sofern es Redoxzentren besitzt, als katalytischer Mediator für die Elektronenübertragung von Enzym oder Coenzym auf die Metallelektrode wirksam werden. Im Falle des Polymethylenblaus läßt sich somit die Höhe des amperometrischen Signals deutlich steigern (Faktor 10), was sich günstig auf das Signal-Rausch-Verhältnis auswirkt und damit zu einer niedrigeren Nachweisgrenze und einer erhöhten Sensitivität für die zu bestimmenden Substanzen führt.

Alternativ können Enzyme oder andere Biokomponenten direkt über reaktive Seitengruppen an den Elektroden immobilisiert werden. Dadurch wird die Konstruktion von Biosensoren mit extrem kurzen Ansprechzeiten im Vergleich zu Membran-Biosensoren ermöglicht, da die Messkomponente nicht durch eine Membran diffundieren muss. Einige Enzyme, wie z.B. Glucoseoxidase, sind Glykoproteine, deren Polysaccharidhülle sich zur chemischen Verknüpfung mit bifunktionellen Spacermolekülen eignet. Diese Spacer werden über eine ihrer beiden funktionellen Gruppen, z.B. über eine Aminofunktion, an die Zuckerreste des Glykoproteins gebunden, während die andere funktionelle Endgruppe, z.B. eine Thiolgruppe, mit der Oberfläche der Goldelektroden eine stabile Verbindung eingeht. Im Falle der Glucoseoxidase wird das Enzymlyophilisat in Carbonatpuffer (pH 8,1) gelöst und nach Zugabe von einprozentiger ethanolischer 2,4-Dinitrofluorbenzollösung mit Natriumperiodatlösung (60 mM) versetzt, worauf eine sogenannte Periodatspaltung des Zuckeranteils des Enzyms erfolgt. Dabei erzeugte reaktive Carbonylgruppen werden im Anschluss an einen dialytischen Reinigungsschritt mit einem bifunktionellen Thiol, z.B. mit Cystamin, über dessen Aminogruppen verkuppelt. Das erhaltene, modifizierte Enzym wird über die Thiolgruppen des Cystaminrestes stabil an die Goldoberfläche gebunden.

Die oben beschriebenen oberflächenporenreichen Mikrostrukturen werden bei der Herstellung von Goldelektroden dadurch erzielt, dass ein chemisch inerter Träger mittels Siebdruck mit einer aus einem Goldpulver und einem polymeren Bindemittel bestehenden Paste beschichtet wird und der beschichtete Träger anschließend durch einen Ofen mit ansteigend abgestuften Brenntemperaturzonen geführt wird, wobei die Temperatur der ersten Brennzone 300°C bis 400°C und die der letzten Brennzone 800°C bis 1100°C beträgt. Die Verweilzeit des beschichteten Trägers beträgt dabei vorzugsweise ca. 3 min bis 8 min.

Mit der Erfindung werden folgende Vorteile erzielt:
Es wurde gefunden, dass die Überspannung für die anodische Oxidation von NADH zu NAD⁺ an den in Dickschichttechnologie mittels Siebdruck hergestellten Edelmetallelektroden derart herabgesetzt wird, daß - im Gegensatz zu herkömmlichen Edelmetallelektroden, die eine Überspannung von +600 mV erforderlich machen - zur amperometrischen Messung von NADH-Konzentrationen Überspannungen von weniger als +200 mV gegenüber einer KCl-gesättigten wäßrigen Kalomelreferenzelektrode (SCE) ausreichend ist. Die Reduktion der Überspannung führt dazu, daß etwaige im flüssigen Analyten vorhandene, bei hohen Überspannungen cooxidierbare Störsubstanzen elektrochemisch nicht mehr umgesetzt werden und das Analysenergebnis nicht mehr verfälschen. Die beschriebenen Edelmetallelektroden finden Verwendung vor allem als Transduktoren für elektrochemische Enzymbiosensoren auf der Basis von Dehydrogenasen. Hauptanwendungsgebiete dieser Sensoren sind die klinische Diagnostik sowie die Umwelt-, Prozeß- und Lebensmittelanalytik.

Im folgenden wird die Erfindung anhand eines Herstellungsbeispiels und anhand von Meßbeispielen näher erläutert.

### Herstellungsbeispiel

Ein Goldpulver mit einer mittleren Teilchengröße von 1,5 µm und einer Größenverteilung von 1 µm bis 3,5 µm wird mit einem Nitrocellulose-haltigen, polymeren Bindemittel innig vermischt. Die so erzeugte Goldpaste wird dann mittels Siebdruck auf eine keramische Trägerplatte der Größe 41 x 41 x 0,5 mm³ aufgetragen. Anschließend wird die beschichtete Trägerplatte mit einer Geschwindigkeit von 8 cm/min durch einen Ofen mit sechs jeweils 60 cm langen Brennzonen mit folgendem Temperaturprofil gefahren:

| | | |
|---|---|---|
| 1. | Zone | 336°C |
| 2. | Zone | 685°C |
| 3. | Zone | 872°C |
| 4. | Zone | 905°C |
| 5. | Zone | 857°C |

Die Teilchengrößenverteilung der Goldpartikel in der verwendeten Siebdruckpaste, sowie deren Radien bestimmen die späteren Oberflächeneigenschaften und die Porosität der Goldelektroden. Zusätzlich führen beim Pyrolysieren des Polymeranteils auftretende Kleinstexplosionen dazu, daß sich scharfkantige Krater auf der Goldoberfläche bilden, die ebenfalls die Oberflächeneigenschaften beeinflussen. Die Kleinstexplosionen entstehen dadurch, daß tieferliegende Polymerschichten so schnell pyrolisieren, daß die Gasdiffusion der darüberliegenden Schichten nicht ausreicht, den entstehenden Druck abzubauen. Mittels vergleichender impedanzspektroskopischer Messungen der tatsächlichen Oberflächengröße herkömmlicher, polierter Goldelektroden und in Dickschichttechnik hergestellter Goldelektroden wurde gezeigt, daß letztere bei gleicher geometrischer Oberfläche eine sechsmal größere tatsächliche Oberfläche besitzen.

### Meßbeispiele gemäß Fig. 1 bis 4

Es zeigen
- Fig. 1: die zyklischen Voltamogramme von (a) einer mikroporösen Dickschichtgoldelektrode und (b) einer kommerziell erhältlichen, hochglanzpolierten, planaren Goldelektrode (EG & G) in gepufferten Lösungen mit (----) und ohne (―) Zusatz von NADH
- Fig. 2: die Langzeitstabilität einer mikroporösen Goldelektrode bei der Messung von NADH
- Fig. 3: den Einfluß von Interferenzsubstanzen bei herkömmlichen und durch die Erfindung ermöglichten Potentialen zur Oxidation von NADH
- Fig. 4: die Konzentrations-Strom-Kennlinie eines Glucose-Biosensors, der mit der erfindungsgemäßen Elektrode als Transduktor bei niedrigen Polarisationsspannungen arbeitet.

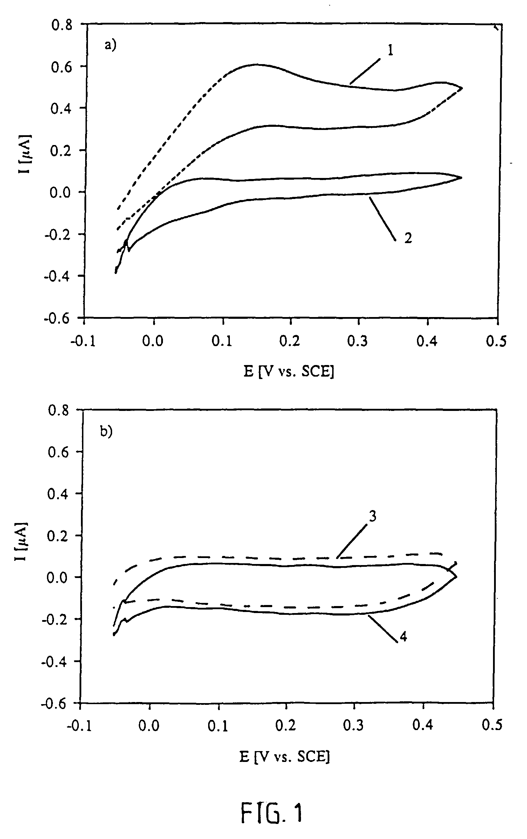

Fig. 1 verdeutlicht die katalytische Aktivität der erfindungsgemäßen Elektroden bezüglich der anodischen Oxidation von NADH. Im Verlaufe des zyklischen Voltamogramms zeigt sich im Falle der Dickschichtgoldelektrode (a) in Gegenwart von NADH (1) bei ca. +100 mV (gegen SCE) beginnend ein deutlicher anodischer Strom, der durch die Oxidation von NADH hervorgerufen wird. Für den NADH-freien Puffer (2) fehlt dieser Strom. Für herkömmliche Elektroden (b) ist ein solches Verhalten unter identischen Versuchsbedingungen nicht feststellbar (3, 4). Die zyklischen Voltamogramme wurden in 0,1 M Natriumphosphatpuffer von pH 7,0, der als Leitsalz 0,1 M Natriumperchlorat enthielt, aufgenommen. Die Voltgeschwindigkeit betrug 10 mV/sec. Die NADH-Konzentration betrug 0 mM (2, 4) bzw. 5 mM (1, 3).

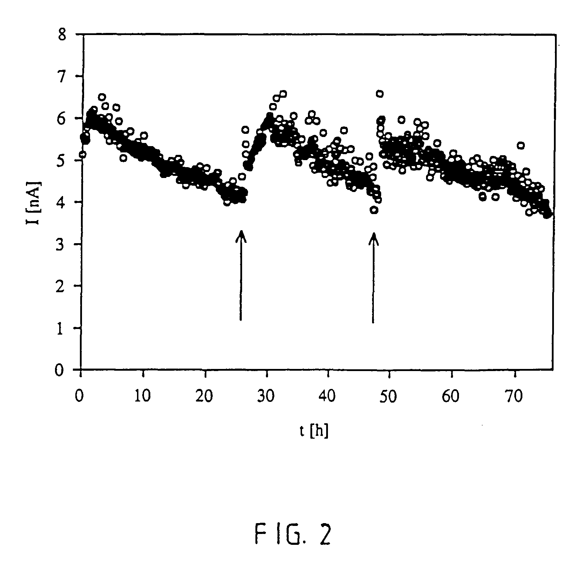

Die erfindungsgemäßen Dickschichtgoldelektroden wurden in ein Fließinjektionssystem zur Detektion von 0,5 mM NADH eingebaut und bei +145 mV (gegen SCE) betrieben. Fig. 2 zeigt den zeitlichen Verlauf des anodischen Stromes für die Oxidation von NADH. Die Pfeile weisen darauf hin, daß jeweils frisch bereitete NADH-Lösungen eingesetzt wurden. Die Abnahme des Stromes nach Einsatz einer frischen Lösung ist somit hauptsächlich eine Folge der thermischen Zersetzung von NADH in Lösung (0,1 M Natriumphosphatpuffer, pH 7,0 mit 0,1 M Natriumperchlorat, Raumtemperatur) und nicht oder nur in geringem Umfang auf Elektrodenfouling zurückzuführen.

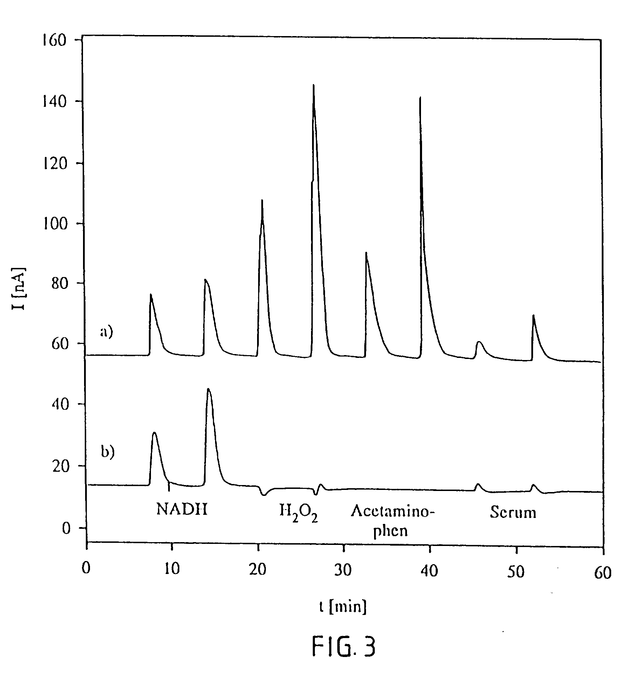

Zur Verdeutlichung der Vorteile eines Sensorbetriebes bei niedrigen Polarisationsspannungen wurden mögliche Interferenzsubstanzen (1 und 2 mM Wasserstoffperoxid, 1 und 2 mM Acetaminophen (= Paracetamol), 1:1 verdünntes und unverdünntes Serum) zusammen mit NADH (1 und 2 mM) in einem Fließinjektionssystem untersucht (Fig. 3). Dabei wurde einmal die bisher gebräuchlichste Spannung von (a) +555 mV an die Elektroden angelegt und (b) einmal bei dem niedrigeren Potential von +145 mV (beide gegen SCE), welches bei den erfindungsgemäßen Elektroden zur Oxidation von NADH ausreicht, gearbeitet. Neben NADH wurden bei diesem niedrigen Potential nur noch Serumkomponenten in geringem Maße angezeigt.

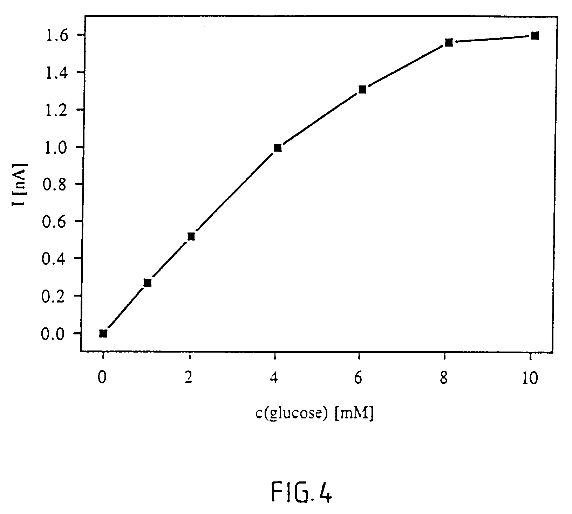

Der Glucose-Biosensor, dessen Eichkurve in Fig. 4 wiedergegeben ist, stellt einen möglichen Anwendungsfall für die erfindungsgemäßen Elektroden dar. Das Enzym Glucose-Dehydrogenase wurde durch Matrixeinschluß in eine Polyvinylacetat-Membran an die Goldoberfläche immobilisiert. Die Eichkurve wurde in einem mit 0,1 M Natriumphosphatpuffer von pH 7,0 betriebenen Fließinjektionssystem aufgenommen, dessen Injektionsvolumen 100 µl bei einer Flußrate von 0,6 ml/min betrug.

## Patentansprüche

1. Elektrochemischer Enzymbiosensor mit Edelmetallelektroden, bei dem eine elektrochemische Oxidation von Pyridinnukleotiden, insbesondere von NADH erfolgt, dadurch gekennzeichnet, dass mindestens eine der Edelmetallelektroden eine oberflächenporenreiche Mikrostruktur mit katalytischen Eigenschaften aufweisen, wodurch die für die elektrochemische Oxidation der Pyridinnukleotide notwendige Überspannung herabgesetzt wird.

2. Elektrochemischer Enzymbiosensor nach Anspruch 1, dadurch gekennzeichnet, dass die Oberfläche einer Elektrode aufgrund der Mikrostrukturen 3 bis 10 mal, vorzugsweise 4 bis 7 mal größer ist, als ihre geometrische Oberfläche.

3. Elektrochemischer Enzymbiosensor nach Anspruch 2, dadurch gekennzeichnet, dass die Elektrodenoberfläche Edelmetallpartikel mit einem mittleren Krümmungsradius von 0,05 bis 4 µm, vorzugsweise von 0,5 µm bis 2 µm, aufweist.

4. Elektrochemischer Enzymbiosensor nach Anspruch 1 - 3, dadurch gekennzeichnet, dass die zur anodischen Oxidation von NADH zu NAD⁺ erforderliche Überspannung auf 0 - 200 mV, vorzugsweise auf 100 bis 150 mV gegen SCE herabgesetzt wird.

5. Elektrochemischer Enzymbiosensor nach Anspruch 1 - 4, dadurch gekennzeichnet, dass die Elektroden aus Gold bestehen.

6. Elektrochemischer Enzymbiosensor nach Anspruch 1 - 5, dadurch gekennzeichnet, dass die Elektroden mit einer Membran beschichtet sind, in der selektivitätsbestimmende Biokomponenten, inbesondere Enzyme immobilisiert sind.

7. Elektrochemischer Enzymbiosensor nach Anspruch 1 - 6, dadurch gekennzeichnet, dass die Elektroden durch Elektropolymerisation, insbesondere von Pyrrol und Methylenblau modifiziert sind .

8. Elektrochemischer Enzymbiosensor nach Anspruch 1 - 7, dadurch gekennzeichnet, dass Enzyme oder andere Biokomponenten direkt über reaktive Seitengruppen an den Elektroden immobilisiert sind.

9. Verfahren zur Herstellung von Edelmetallelektroden für einen elektrochemischen Biosensor gemäß den Ansprüchen 1 - 6, dadurch gekennzeichnet, dass ein chemisch inerter Träger mittels Siebdruck mit einer aus einem Goldpulver und einem polymeren Bindemittel bestehenden Paste beschichtet wird und der beschichtete Träger durch einen Ofen mit ansteigend abgestuften Brenntemperaturzonen geführt wird, wobei die Temperatur der ersten Brennzone 300°C bis 400°C und die der letzten Brennzone 800°C bis 1100°C beträgt.

10. Verfahren nach Anspruch 9, dadurch gekennzeichnet, dass die Verweilzeit des beschichtenen Trägers dabei von 3 min bis 8 min beträgt.

## Claims

1. Electrochemical enzyme biosensor which comprises noble metal electrodes and with which an electrochemical oxidation of pyridine nucleotides, in particular of NADH takes place, characterized in that at least one of the noble metal electrodes has a microtexture rich in surface pores and having catalytic properties, as a result of which the overpotential required for electrochemical oxidation of the pyridine nucleotides is reduced.

2. Electrochemical enzyme biosensor according to Claim 1, characterized in that, owing to the microtextures, the surface area of an electrode is from 3 to 10 times, preferably from 4 to 7 times, larger than its geometric surface area.

3. Electrochemical enzyme biosensor according to Claim 2, characterized in that the electrode surface comprises noble metal particles having a mean radius of curvature of from 0.05 to 4 µm, preferably from 0.5 µm to 2 µm.

4. Electrochemical enzyme biosensor according to Claim 1 - 3, characterized in that the overpotential required for the anodic oxidation of NADH to NAD⁺ is reduced to 0 - 200 mV, preferably to from 100 to 150 mV, against the SCE.

5. Electrochemical enzyme biosensor according to Claim 1 - 4, characterized in that the electrodes are made of gold.

6. Electrochemical enzyme biosensor according to Claim 1 - 5, characterized in that the electrodes are coated with a membrane, in which selectivity-determining biocomponents, in particular enzymes, are immobilized.

7. Electrochemical enzyme biosensor according to Claim 1 - 6, characterized in that the electrodes have been modified by electropolymerization, in particular of pyrrole and methylene blue.

8. Electrochemical enzyme biosensor according to Claim 1 - 7, characterized in that enzymes or other biocomponents are immobilized directly on the electrodes via reactive pendent groups.

9. Method for fabricating noble metal electrodes for an electrochemical biosensor according to Claims 1 - 6, characterized in that a chemically inert base is coated, by means of screen printing, with a paste comprising a gold powder and a polymeric binder, and the coated base is passed through a furnace having incrementally gradated firing temperature zones, the temperature of the first firing zone being from 300°C to 400°C and that of the last firing zone being from 800°C to 1100°C.

10. Method according to Claim 9, characterized in that the residence time of the coated base is in this case from 3 min to 8 min.

## Revendications

1. Biocapteur enzymatique électrochimique à électrodes de métal noble, dans lequel s'effectue une oxydation électrochimique de pyridine - nucléotides, en particulier de NADH, caractérisé en ce qu'au moins une des électrodes de métal noble présente une microstructure riche en pores superficiels douée de propriétés catalytiques, ce qui a pour effet que la surtension nécessaire pour l'oxydation électrochimique des pyridine - nucléotides est réduite.

2. Biocapteur enzymatique électrochimique suivant la revendication 1, caractérisé en ce que la surface d'une électrode sur la base des microstructures est 3 à 10 fois supérieure, de préférence 4 à 7 fois supérieure à sa surface géométrique.

3. Biocapteur enzymatique électrochimique suivant la revendication 2, caractérisé en ce que la surface des électrodes présente des particules de métal noble ayant un rayon moyen de courbure de 0,05 à 4 µm, de préférence de 0,5 µm à 2 µm.

4. Biocapteur enzymatique électrochimique suivant les revendications 1 à 3, caractérisé en ce que la surtension nécessaire pour l'oxydation anodique de NADH en NAD⁺ est réduite à 0 - 200 mV, de préférence à 100 - 150 mV par rapport à l'électrode de référence au calomel.

5. Biocapteur enzymatique électrochimique suivant les revendications 1 à 4, caractérisé en ce que les électrodes sont en or.

6. Biocapteur enzymatique électrochimique suivant les revendications 1 à 5, caractérisé en ce que les électrodes sont revêtues d'une membrane dans laquelle sont immobilisés des biocomposants déterminant la sélectivité, en particulier des enzymes.

7. Biocapteur enzymatique électrochimique suivant les revendications 1 à 6, caractérisé en ce que les électrodes sont modifiées par électropolymérisation, notamment du pyrrole et du bleu de méthylène.

8. Biocapteur enzymatique électrochimique suivant les revendications 1 à 7, caractérisé en ce que des enzymes ou d'autres biocomposants sont immobilisés directement sur les électrodes au moyen de groupes latéraux réactifs.

9. Procédé de production d'électrodes en métal noble pour un biocapteur électrochimique suivant les revendications 1 à 6, caractérisé en ce qu'un support chimiquement inerte est revêtu par sérigraphie d'une pâte constituée de poudre d'or et d'un liant polymère et le support revêtu est amené à traverser un four présentant des zones de cuisson à échelonnement croissant des températures, la température de la première zone de cuisson allant de 300°C à 400°C et celle de la dernière zone de cuisson allant de 800°C à 1100°C.

10. Procédé suivant la revendication 9, caractérisé en ce que la durée de séjour dans le four du support revêtu va de 3 minutes à 8 minutes.
